Europäisches Patentamt

European Patent Office

Office européen des brevets

(11) Publication number: **0 322 147 B1**

# EUROPEAN PATENT SPECIFICATION

(45) Date of publication of patent specification: **06.05.92**  (51) Int. Cl.⁵: **C07C 2/72**

(21) Application number: **88311795.4**

(22) Date of filing: **13.12.88**

(54) **Coupling process.**

(30) Priority: **21.12.87 US 135318**

(43) Date of publication of application:
**28.06.89 Bulletin 89/26**

(45) Publication of the grant of the patent:
**06.05.92 Bulletin 92/19**

(84) Designated Contracting States:
**AT BE CH DE ES FR GB GR IT LI LU NL SE**

(56) References cited:
**EP-A- 0 128 001**

(73) Proprietor: **ETHYL CORPORATION**
**Ethyl Tower 451 Florida Boulevard**
**Baton Rouge Louisiana 70801(US)**

(72) Inventor: **Smith, Robert Scott**
**8978 G.S.R.I. Avenue**
**Baton Rouge Louisiana 70810(US)**

(74) Representative: **Collier, Jeremy Austin Grey et al**
**J.A.Kemp & Co. 14, South Square Gray's Inn**
**London WC1R 5EU(GB)**

Rank Xerox (UK) Business Services
(3.08/2.18/2.0)

## Description

This invention relates to a process for coupling an ethylenically-unsaturated aliphatic hydrocarbon with an aromatic hydrocarbon having an active hydrogen on a saturated alpha-carbon.

As disclosed, e.g., in U.S. Patent 4,179,580 (Cobb), European Specification 128001A (Kudoh et al.), and Eberhardt et al., Journal of Organic Chemistry, Vol. 30, pp. 82-84 (1965), it is known that supported alkali metals are useful as catalysts in the coupling of ethylenically-unsaturated hydrocarbons with aromatic hydrocarbons having an active hydrogen on a saturated alpha-carbon. For example, European Specification 128001A discloses the use of a sodium catalyst on a carrier of the formula $K_2O. x Al_2O_3$ where x is 0.5 to 11. The supported alkali metals are more effective than the corresponding unsupported alkali metals in such reactions but are still not as effective as might be desired.

Claff et al., Journal of Organic Chemistry, Vol. 20, pp. 440-442 and 981-986 (1955) disclose the use of sodium oxide in the metalation of toluene by potassium.

As object of this invention is to provide a novel process for coupling an ethylenically-unsaturated aliphatic hydrocarbon with an aromatic hydrocarbon having an active hydrogen on a saturated alpha-carbon.

Another object is to provide such a process which utilizes a supported alkali metal as a catalyst.

A further object is to provide such a process in which the reaction rate and product yield are increased.

These and other objects are attained by coupling an ethylenically-unsaturated aliphatic hydrocarbon with an aromatic hydrocarbon having an active hydrogen on a saturated alpha-carbon in the presence of a supported alkali metal as a catalyst and 10-100 mol %, based on the amount of the alkali metal catalyst, of sodium oxide or potassium oxide as a co-catalyst.

The ethylenically-unsaturated aliphatic hydrocarbon which is coupled with the aromatic hydrocarbon in the practice of the invention may be any of the ethylenically-unsaturated aliphatic hydrocarbons that are known to be useful in such reactions, such as ethene, propene, 1-butene, 2-butene, isobutene, 1-pentene, 2-pentene, 3-methyl-1-butene, 2-methyl-2-butene, 1-hexene, 2-hexene, 3-hexene, 4-methyl-1-pentene, 3-methyl-1-pentene, 4-methyl-2-pentene, 1-heptene, 2-heptene, 2-octene, 4-nonene, 1-decene, 2-decene, 1-dodecene, 3-tetradecene, 5-hexadecene, 6-methyl-4-heptadecene, 1-eicosene, butadiene, isoprene, and 2,5-dimethyl-1,5-hexadiene. However, it is generally a hydrocarbon corresponding to the formula $QQ'C=CTT'$, in which Q, Q', T, and T' are independently selected from hydrogen and alkyl and alkenyl groups of up to 20 carbons; and it is apt preferably to be an alkene of up to 20 carbons. Particularly preferred ethylenically-unsaturated hydrocarbons are ethene and propene.

The aromatic hydrocarbon having an active hydrogen on a saturated alpha-carbon may be any such compound that is known to be useful in such reactions, such as toluene, ethylbenzene, n-propylbenzene, isopropylbenzene, n-butylbenzene, sec-butylbenzene, isobutylbenzene, n-eicosylbenzene, o-, m-, and p-xylenes, o-, m-, and p-ethyltoluenes, 1,3,5-trimethylbenzene, 1,2,4,5- and 1,2,3,5-tetramethylbenzenes, p-diisopropylbenzene, 1- and 2-methylnaphthalenes, dimethylnaphthalenes, 1-ethyl-4-n-octadecylnaphthalene, 1,4-di-n-pentylnaphthalene, 1,2,3,4-tetrahydronaphthalene, indan, cyclohexylbenzene, methylcyclohexylbenzene, and diphenylmethane. However, it is generally a hydrocarbon corresponding to the formula $RR'R''CH$, in which R is an aryl group of up to 20 carbons and R' and R'' are independently selected from hydrogen and alkyl and aryl groups of up to 20 carbons; and it is apt preferably to be an alkylbenzene having one or more aralkyl groups. A particularly preferred aromatic hydrocarbon is toluene.

The mol ratio of ethylenically-unsaturated hydrocarbon to aromatic hydrocarbon varies with the particular reactants employed and the products desired, particularly since the aromatic hydrocarbon may have one or more active hydrogens, and it may be desired to react the ethylenically-unsaturated hydrogen with only one or with more than one active hydrogen in the aromatic hydrocarbon. It is frequently preferred to employ the reactants in the stoichiometric amounts appropriate for the preparation of the desired product. However, either reactant can be used in excess.

As in Cobb, the alkali metal employed as a catalyst may be lithium, sodium, potassium, rubidium, or cesium; and it appropriately has its surface area increased by being finely divided or liquid as well as by being supported on any suitable support material, such as diatomaceous earth, activated charcoal, granular coke, silicon, pumice, porcelain, quartz, steel turnings, copper shot, sodium carbonate, and potassium carbonate. However, it is preferably potassium or a potassium alloy, e.g., a sodium/potassium alloy. The amount of alkali metal used is a catalytic amount, generally 2-10 mol %, based on the amount of either of the reactants when they are employed in equimolar amounts or on the amount of the major reactant when they are not utilized in equimolar amounts.

The co-catalyst of the invention is an oxide of sodium or potassium. It is preferably sodium oxide, since potassium oxide is dangerous to make and is therefore less likely to be available. Like the alkali metal, it is used in finely divided form; and it may be incorporated into the reaction mixture as the oxide, or it may be generated in situ, e.g., by oxidizing supported sodium or potassium. The amount of co-catalyst used is 10-100 mol %, based on the amount of the alkali metal catalyst.

The reaction is conducted by heating a mixture of the ethylenically-unsaturated aliphatic hydrocarbon, the active hydrogen-containing aromatic hydrocarbon, the supported catalyst, and the co-catalyst under substantially anhydrous conditions at a suitable temperature, generally 100-300°C., preferably 175-200., to couple the reactants. It is generally conducted in the absence of a diluent or in the presence of an excess of the active hydrogen-containing aromatic hydrocarbon as the sole diluent. However, an inert diluent can be used if desired. Exemplary of such diluents are liquid alkanes, cycloalkanes, and aromatic hydrocarbons, such as pentane, hexane, isooctane, cyclohexane, naphthalene, decahydronaphthalene, and white oils.

The process of the invention proceeds at a faster rate and provides higher product yields with fewer by-products than comparable processes conducted in the absence of the co-catalyst, and it is particularly advantageous as a means of alkylating alkylaromatic compounds, especially alkylbenzenes, to form compounds useful in various applications such as solvents, internal standards, intermediates for polymers, pharmaceuticals, or pesticides.

The following examples are given to illustrate the invention:

## COMPARATIVE EXAMPLE A

A suitable reaction vessel was charged with 4.9g of diatomaceous earth, 92g (1.0 mol) of toluene, $C_{11}$ paraffin as a GC standard, and 1.0g of NaK (an alloy having a K content of 78% by weight). The mixture was stirred and heated to 185°C in 15 minutes, after which propene was charged until a pressure of 2758 kPa was reached; and the propene pressure was then maintained at 2689-2758 kPa throughout the reaction. Periodically the stirrer was stopped to allow the solids to settle; and samples were drawn, allowed to cool to room temperature, and subjected to GC analysis to determine the amounts of unreacted toluene, desired isobutylbenzene (IBB) product, and n-butylbenzene (NBB), methylindan (MI), and methylpentene (MP) by-products. Finally the reaction was stopped by cooling the reactor to 75°C, venting most of the propene, and injecting 10 mL of methanol under nitrogen pressure to quench the catalyst. The results of the analyses are shown below.

| Time (min.) | Mols x 100 | | | | |
|---|---|---|---|---|---|
| | Toluene | IBB | NBB | MI | MP |
| 0 | 100 | 0 | 0 | 0 | 0 |
| 40 | 100 | 0 | 0 | 0 | 0 |
| 80 | 93.0 | 3.5 | 0.2 | 0.46 | 0.4 |
| 120 | 78.3 | 11.3 | 0.53 | 2.56 | 1.2 |
| 160 | 66.0 | 17.4 | 0.81 | 4.12 | 1.7 |
| 200 | 62.1 | 21.2 | 0.98 | 4.90 | 2.0 |
| 240 | 60.3 | 22.8 | 1.05 | 5.18 | 2.2 |

## COMPARATIVE EXAMPLE B

Comparative Example A was repeated except that the diatomaceous earth support was replaced by 6.6g of sodium oxide having a particle size of -325 mesh. The analytical results are shown below.

| Time (min.) | Mols x 100 | | | | |
|---|---|---|---|---|---|
| | Toluene | IBB | NBB | MI | MP |
| 0 | 100 | 0 | 0 | 0 | 0 |
| 40 | 91.9 | 8.8 | 1.11 | 0.06 | 0.6 |
| 80 | 83.0 | 13.1 | 1.60 | 0.09 | 0.9 |
| 160 | 72.2 | 22.1 | 2.45 | 0.17 | 1.6 |

EXAMPLE I

Comparative Example A was repeated except that 2.2 mols of sodium oxide having a particle size of -325 mesh was mixed with the diatomaceous earth and ground together with it prior to charging them to the reaction vessel. The analytical results are shown below.

| Time (min.) | Mols x 100 | | | | |
|---|---|---|---|---|---|
| | Toluene | IBB | NBB | MI | MP |
| 0 | 100 | 0 | 0 | 0 | 0 |
| 80 | 84.0 | 5.2 | 0.57 | 0.12 | 0.5 |
| 120 | 72.0 | 20.8 | 2.25 | 0.39 | 2.4 |
| 160 | 50.4 | 37.5 | 4.17 | 0.62 | 4.4 |
| 200 | 39.8 | 49.7 | 5.14 | 0.73 | 6.0 |
| 240 | 29.5 | 57.2 | 5.57 | 0.82 | 7.1 |

EXAMPLE II

Example I was repeated except that the amount of sodium oxide was increased to 4.3 mmols. The analytical results are shown below.

| Time (min.) | Mols x 100 | | | | |
|---|---|---|---|---|---|
| | Toluene | IBB | NBB | MI | MP |
| 0 | 100 | 0 | 0 | 0 | 0 |
| 80 | 90.2 | 7.8 | 0.95 | 0.13 | 0.8 |
| 120 | 71.7 | 23.9 | 2.95 | 0.35 | 2.7 |
| 160 | 50.8 | 41.0 | 4.83 | 0.48 | 4.9 |
| 200 | 35.8 | 53.3 | 5.89 | 0.56 | 7.0 |
| 240 | 27.4 | 60.5 | 6.30 | 0.61 | 8.9 |

EXAMPLE III

Example I was repeated except that the amount of sodium oxide was increased to 8.6 mmols. The analytical results are shown below.

| Time (min.) | Mols x 100 | | | | |
|---|---|---|---|---|---|
| | Toluene | IBB | NBB | MI | MP |
| 0 | 100 | 0 | 0 | 0 | 0 |
| 80 | 84.9 | 9.3 | 1.13 | 0.13 | 1.1 |
| 120 | 68.2 | 25.5 | 3.22 | 0.30 | 2.8 |
| 160 | 48.1 | 42.5 | 5.09 | 0.35 | 5.5 |
| 200 | 33.3 | 54.1 | 6.10 | 0.38 | 7.6 |
| 240 | 25.0 | 61.1 | 6.45 | 0.43 | 9.6 |

EXAMPLE IV

Example III was repeated except that the amount of NaK used was 1.3g. The analytical results are shown below.

| Time (min.) | Mols x 100 | | | | |
|---|---|---|---|---|---|
| | Toluene | IBB | NBB | MI | MP |
| 0 | 100 | 0 | 0 | 0 | 0 |
| 80 | 87.9 | 11.5 | 1.44 | 0.14 | 0.9 |
| 120 | 65.2 | 29.4 | 3.78 | 0.31 | 3.0 |
| 160 | 46.5 | 47.5 | 5.85 | 0.35 | 5.7 |
| 200 | 27.9 | 60.7 | 6.96 | 0.35 | 8.4 |
| 240 | 18.6 | 69.0 | 7.35 | 0.35 | 11.5 |

EXAMPLE V

Example III was repeated except that the sodium oxide employed had a particle size of +80 to -325 mesh. The analytical results are shown below.

| Time (min.) | Mols x 100 | | | | |
|---|---|---|---|---|---|
| | Toluene | IBB | NBB | MI | MP |
| 0 | 100 | 0 | 0 | 0 | 0 |
| 80 | 84.0 | 9.1 | 0.94 | 0.28 | 0.8 |
| 120 | 66.6 | 24.7 | 2.71 | 0.56 | 2.2 |
| 160 | 53.4 | 37.0 | 3.81 | 0.67 | 3.5 |
| 200 | 42.3 | 45.7 | 4.46 | 0.80 | 4.3 |
| 240 | 36.0 | 51.4 | 4.76 | 0.91 | 5.2 |

EXAMPLE VI

Example III was repeated except that the NaK was replaced with 1.0g of potassium. The analytical results are shown below.

| Time (min.) | Mols x 100 | | | | |
|---|---|---|---|---|---|
| | Toluene | IBB | NBB | MI | MP |
| 0 | 100 | 0 | 0 | 0 | 0 |
| 40 | 99.6 | 1.7 | 0.18 | 0 | 0.1 |
| 80 | 85.6 | 11.5 | 1.48 | 0.15 | 1.2 |
| 120 | 63.1 | 29.0 | 3.76 | 0.32 | 3.1 |
| 160 | 43.4 | 46.1 | 5.68 | 0.36 | 5.3 |
| 200 | 30.1 | 57.8 | 6.61 | 0.38 | 7.5 |
| 240 | 21.5 | 63.4 | 6.94 | 0.42 | 9.2 |

## EXAMPLE VII

Example III was repeated except that the propene pressure was about 2069 kPa. The analytical results are shown below.

| Time (min.) | Mols x 100 | | | | |
|---|---|---|---|---|---|
| | Toluene | IBB | NBB | MI | MP |
| 0 | 100 | 0 | 0 | 0 | 0 |
| 120 | 80.5 | 18.0 | 2.29 | 0.20 | 1.0 |
| 160 | 64.9 | 33.4 | 4.13 | 0.26 | 1.8 |
| 200 | 47.6 | 45.8 | 5.40 | 0.31 | 2.8 |
| 240 | 39.3 | 52.9 | 5.89 | 0.37 | 3.4 |

## EXAMPLE VIII

Example III was repeated except that the reaction temperature was 165°C. The analytical results are shown below.

| Time (min.) | Mols x 100 | | | | |
|---|---|---|---|---|---|
| | Toluene | IBB | NBB | MI | MP |
| 0 | 100 | 0 | 0 | 0 | 0 |
| 120 | 97.7 | 3.4 | 0.38 | 0 | 0.4 |
| 160 | 93.9 | 7.4 | 0.90 | 0.06 | 0.9 |
| 200 | 86.2 | 13.4 | 1.70 | 0.14 | 1.9 |
| 240 | 77.1 | 20.5 | 2.63 | 0.21 | 2.9 |

## Claims

1. A process for coupling an ethylenically-unsaturated aliphatic hydrocarbon with an aromatic hydrocarbon having an active hydrogen on a saturated alpha-carbon by contacting the said unsaturated aliphatic hydrocarbon with the said aromatic hydrocarbon in the presence of a supported alkali metal as catalyst, characterized in that the contact is conducted in the presence of 10-100 mol %, based on the alkali metal catalyst, of sodium oxide or potassium oxide as co-catalyst.

2. The process of claim 1 wherein the ethylenically-unsaturated aliphatic hydrocarbon is a hydrocarbon corresponding to the formula $QQ'C = CTT'$, in which $Q$, $Q'$, $T$, and $T'$ are each hydrogen or alkyl or alkenyl groups of up to 20 carbons each.

6

3. The process of claim 2 wherein the ethylenically-unsaturated aliphatic hydrocarbon is an alkene of 2-20 carbons.

4. The process of any one of claims 1 to 3 wherein the aromatic hydrocarbon is a hydrocarbon corresponding to the formula $RR'R''CH$, in which R is an aryl group of up to 20 carbons and $R'$ and $R''$ are each hydrogen or alkyl or aryl groups of up to 20 carbons each.

5. The process of claim 4 wherein the aromatic hydrocarbon is an alkylbenzene.

6. The process of any one of claims 1 to 5 wherein the alkali metal catalyst is potassium or a potassium alloy.

7. The process of any one of claims 1 to 6 wherein the co-catalyst is sodium oxide.

8. The process of any one of claims 1 to 7 which is conducted at a temperature of 100-300°C.

9. The process of claim 1 wherein propene is coupled with toluene at 175-200°C in the presence of a supported potassium or sodium/potassium catalyst and an oxide of sodium or of potassium as co-catalyst.

**Revendications**

1. Procédé de couplage d'un hydrocarbure aliphatique à non-saturation éthylénique avec un hydrocarbure aromatique portant un atome actif d'hydrogène sur un atome de carbone alpha saturé par mise en contact dudit hydrocarbure aliphatique non saturé avec ledit hydrocarbure aromatique en présence d'un catalyseur constitué d'un métal alcalin fixé sur un support, caractérisé en ce que le contact est établi en présence de 10 à 100 moles %, sur la base du catalyseur au métal alcalin, d'oxyde de sodium ou d'oxyde de potassium comme co-catalyseur.

2. Procédé suivant la revendication 1, dans lequel l'hydrocarbure aliphatique à non-saturation éthylénique est un hydrocarbure correspondant à la formule $QQ'C = CTT'$ dans laquelle Q, Q', T et T' représentent chacun l'hydrogène ou des groupes alkyle ou alcényle ayant jusqu'à 20 atomes de carbone chacun.

3. Procédé suivant la revendication 2, dans lequel l'hydrocarbure aliphatique à non-saturation éthylénique est un alcène de 2 à 20 atomes de carbone.

4. Procédé suivant l'une quelconque des revendications 1 à 3, dans lequel l'hydrocarbure aromatique est un hydrocarbure correspondant à la formule $RR'R''CH$ dans laquelle R est un groupe aryle ayant jusqu'à 20 atomes de carbone et R' et R'' représentent chacun de l'hydrogène ou des groupes alkyle ou aryle ayant jusqu'à 20 atomes de carbone chacun.

5. Procédé suivant la revendication 4, dans lequel l'hydrocarbure aromatique est un alkylbenzène.

6. Procédé suivant l'une quelconque des revendications 1 à 5, dans lequel le catalyseur au métal alcalin est le potassium ou un alliage de potassium.

7. Procédé suivant l'une quelconque des revendications 1 à 6, dans lequel le co-catalyseur est l'oxyde de sodium.

8. Procédé suivant l'une quelconque des revendications 1 à 7, qui est mis en oeuvre à une température de 100 à 300°C.

9. Procédé suivant la revendication 1, dans lequel du propène est couplé à du toluène à 175-200°C en présence d'un catalyseur au potassium ou au sodium/potassium fixé sur un support et d'un co-catalyseur consistant en oxyde de sodium ou en oxyde de potassium.

**Patentansprüche**

1. Verfahren zur Kupplung eines ethylenisch ungesättigten aliphatischen Kohlenwasserstoffs an einen aromatischen Kohlenwasserstoff mit einem aktiven Wasserstoffatom an einem gesättigten α-Kohlenstoffatom durch In-Kontakt-Bringen des ungesättigten aliphatischen Kohlenwasserstoffs mit dem aromatischen Kohlenwasserstoff in Gegenwart eines Träger-gestützten Alkalimetalls als Katalysator, dadurch gekennzeichnet, daß das In-Kontakt-Bringen in Gegenwart von 10 bis 100 Molprozent Natriumoxid oder Kaliumoxid als Co-Katalysator, bezogen auf den Alkalimetall-Katalysator durchgeführt wird.

2. Verfahren nach Anspruch 1, worin der ethylenisch ungesättigte aliphatische Kohlenwasserstoff ein Kohlenwasserstoff ist, der der Formel QQ'C = CTT' entspricht, in der Q, Q', T und T' jeweils Wasserstoff oder Alkylgruppen oder Alkenylgruppen mit jeweils bis zum 20 Kohlenstoffatomen sind.

3. Verfahren nach Anspruch 2, worin der ethylenisch ungesättigte aliphatische Kohlenwasserstoff ein Alken mit 2 bis 20 Kohlenstoffatomen ist.

4. Verfahren nach irgendeinem der Ansprüche 1 bis 3, worin der aromatische Kohlenwasserstoff ein Kohlenwasserstoff ist, der der Formel RR'R''CH entspricht, worin R eine Arylgruppe mit bis zu 20 Kohlenstoffatomen ist und R' und R'' jeweils Wasserstoff oder Alkylgruppen oder Arylgruppen mit jeweils bis zu 20 Kohlenstoffatomen sind.

5. Verfahren nach Anspruch 4, worin der aromatische Kohlenwasserstoff ein Alkylbenzol ist.

6. Verfahren nach irgendeinem der Ansprüche 1 bis 5, worin der Alkalimetall-Katalysator Kalium oder eine Kalium-Legierung ist.

7. Verfahren nach irgendeinem der Ansprüche 1 bis 6, worin der Co-Katalysator Natriumoxid ist.

8. Verfahren nach irgendeinem der Ansprüche 1 bis 7, welches bei einer Temperatur von 100 bis 300°C durchgeführt wird.

9. Verfahren nach Anspruch 1, worin Propen mit Toluol bei 175 bis 200°C in Gegenwart eines Träger-gestützten Kalium-oder Natrium/Kalium-Katalysator und eines Oxids von Natrium oder von Kalium als Co-Katalysator gekuppelt wird.